# EUROPÄISCHE PATENTSCHRIFT

(11) **EP 1 094 999 B1**
(45) Veröffentlichungstag und Bekanntmachung des Hinweises auf die Patenterteilung: **15.09.2004**
(21) Anmeldenummer: 99936463.1
(22) Anmeldetag: 29.06.1999
(51) Int. Cl.: C07C 17/389, C07C 51/47, C07C 51/64, C07C 59/21

(54) **HERSTELLUNG VON FLUORIDARMEN ORGANISCHEN VERBINDUNGEN**
PRODUCTION OF ORGANIC COMPOUNDS WHICH ARE LOW IN FLUORIDE
COMPOSES ORGANIQUES A FAIBLE TENEUR EN FLUORURE

(30) Priorität: 06.07.1998 DE 19829909; 30.10.1998 DE 19850010
(43) Veröffentlichungstag der Anmeldung: 02.05.2001
(73) Patentinhaber: Solvay Fluor und Derivate GmbH, 30173 Hannover (DE)
(72) Erfinder: BRAUN, Max, D-30900 Wedemark (DE); EICHER, Johannes, D-74229 Oedheim (DE); JANSSENS, Francine, B-1800 Vilvoorde (BE); EICHHOLZ, Kerstin, D-30855 Langenhagen (DE); PALSHERM, Stefan, D-30890 Barsinghausen (DE)
(74) Vertreter: Gosmann, Martin, Dr.
(86) Internationale Anmeldenummer: PCT/EP1999/004477
(87) Internationale Veröffentlichungsnummer: WO 2000/001647

(56) Entgegenhaltungen:
- EP-A- 0 566 974
- EP-A- 0 623 582
- WO-A-90/11270
- DE-A- 2 044 986
- DE-A- 3 311 751
- DE-B- 1 158 490
- DE-C- 19 732 031
- DATABASE REGISTRY [Online] ACS Field RN, XP002127114

## Beschreibung

Die Erfindung bezieht sich auf ein Verfahren zur katalysierten Herstellung von fluoridarmen organischen Verbindungen, insbesondere fluororganischen Verbindungen wie Carbonsäuren und ihren Derivaten.

Fluororganische Verbindungen haben in vielen Gebieten der Technik große Bedeutung. Auf dem Gebiet der Kältetechnik, im Feuerlöschwesen, als Reinigungsmittel werden beispielsweise fluororganische Verbindungen eingesetzt (Fluorkohlenwasserstoffe, Fluorchlorkohlenwasserstoffe, fluorhaltige Ether, fluorhaltige Tenside etc.). Als Treibmittel zur Herstellung von Schäumen oder Aerosolen (auch im Bereich der pharmazeutischen Galenik) werden Fluorkohlenwasserstoffe eingesetzt. Fluororganische Verbindungen werden nicht nur, wie oben erläutert, als Endprodukte, sondern auch als Zwischenprodukte für die Herstellung nützlicher Weiterverarbeitungsprodukte verwendet. Insbesondere im Gebiet der Agrotechnik haben z. B. Fluor und gegebenenfalls Chlor enthaltende Carbonsäuren als Baustein großes Interesse gefunden. Derartige Carbonsäuren und reaktive Derivate wie entsprechende Carbonsäurechloride können - oft in Kondensationsreaktionen - zu interessanten Bausteinen weiterverarbeitet werden, beispielsweise zu Estern oder Ringsystemen.

Viele Carbonsäuren und Ester von Carbonsäuren werden in der Technik als solche verwendet. Essigester und andere Carbonsäureester dienen beispielsweise als Lösungs- oder Reinigungsmittel, andere Ester, z. B. von Bernsteinsäure, werden zur Aromatisierung eingesetzt. Der Trifluoressigsäureethylester ist beispielsweise ein Lösungsmittel für die Chlorierung von Paraffinen oder die Polymerisation von Olefinoxiden. Viele Carbonsäureester sind auch Zwischenprodukte in der chemischen Synthese. Der Trifluoressigsäuremethylester und Trifluoressigsäure-1.1.1-Trifluorethylester liefern nach Hydrierung Trifluorethanol (und ggf. Methanol). Trifluorethanol wird als Lösungsmittel und als Zwischenprodukt, beispielsweise bei der Herstellung des Lösungsmittels und Anästhetikums Isoflurane, verwendet. Ester der Trifluoressigsäure und Trifluoracetessigsäure dienen auch zur Einführung bzw. zur Herstellung von biologisch wirksamen Verbindungen, die eine CF₃-Gruppe aufweisen. Beispielsweise kann man durch N-Acylierung mit Trifluoressigsäuremethylester Peptide mit hormonaler Aktivität herstellen. Der Trifluorethylester liefert mit Kampfer-Derivaten Shiftreagenzien für die NMR-Analyse. Der Trifluoressigsäurephenylester liefert nach Fries'scher Verschiebung mit Aluminiumchlorid das entsprechende trifluoracetylierte Phenol, welches ein Synthesebaustein für Pharmazeutika ist. Viele weitere Anwendungszwecke von Estern sind dem Fachmann bekannt, beispielsweise die Umsetzung von Estern mit Aminen zu Amiden, die Synthesebausteine für Pharmazeutika, Photosensibilisatoren und Farbstoffe darstellen.

Ester der Chlordifluoressigsäure sind ebenfalls Synthesebausteine. Der Ethylester dient beispielsweise zum Aufbau von Flüssigkristallen, siehe DE-OS 40 23 106, bei der Herstellung von Arzneimitteln, siehe US-A 5,006,563, der Methylester ebenfalls für den Aufbau von Flüssigkristallen, als Ausgangsverbindung für die mikrobielle Herstellung chiraler sekundärer Alkohole, siehe T. Kitazume et al., J. Fluorine Chem. 56 (1992), Seiten 271 bis 284, oder für die Herstellung fluorierter Enolether in der Wittig-Synthese, siehe J. P. Beque et al., J. Org. Chem. 57 (1992), Seite 3807 ff. Die Ester der Chlordifluoressigsäure sind auch Vorstufen für Difluorcarben.

Carbonsäurechloride, insbesondere Fluor enthaltende Carbonsäurechloride, werden mit Keten zu Verbindungen des Typs RC(O)CH₂C(O)Cl umgesetzt, die verestert werden und ebenfalls Synthesebausteine sind. Die Herstellung von Halogenacetessigsäurechloriden und ihre Veresterung offenbart die DE-AS 1 158 490. Die Ester sind Zwischenprodukte in der Farbstoffchemie, pharmazeutischen Chemie und Pflanzenschutzchemie.

Fluororganische Verbindungen können beispielsweise mittels direkter Fluorierung mit F₂, höhervalehten Metallfluoriden, durch Chlor-Fluoraustauch, HF-Addition und andere Verfahren hergestellt werden. Sofern hydrolysierbares Fluorid, sei es herstellungsbedingt oder durch Hydrolyse von Fluor aus dem Molekül, enthalten ist, kann dies zu Korrosionsproblemen mit Glas-, Keramik- und Metallbehältnissen oder Apparaturen führen.

Fluorwasserstoff reagiert mit Glas und Keramik schließlich unter Bildung von H₂SiF₆, die ihrerseits unter bestimmten Bedingungen mit Wasser wieder HF und SiO₂ bildet. Das freigesetzte HF greift wiederum Glas an, so daß große Schäden entstehen können, Korrosion an Metallbehältern ist ebenfalls unerwünscht.

Nicht nur in Vorratsbehältern, sondern auch bei der Derivatisierung von fluororganischen Verbindungen treten Probleme auf, z. B. bei Kondensationsreaktionen, bei denen HF - sei es als Sekundärprodukt - freigesetzt wird.

Die Veresterung von Carbonsäurechloriden ohne Katalysator mit Alkoholen führt z. B. zu Korrosionsproblemen, sofern die Carbonsäurechloride herstellungsbedingt geringe Mengen Carbonsäurefluoride, freie HF, oder hydrolysierbares Fluorid aufweisen. Auch in Carbonsäuren bzw. deren Estern stört oft die genannte Verunreinigung, auch wegen Korrosionsproblemen.

Die deutsche Offenlegungsschrift DE 33 11 751 A1 offenbart ein Verfahren zur Reinigung von Fluorkohlenwasserstoffen. Verwendet werden Zeolithe, mit denen Halogene und anorganische Halogenverbindungen abgetrennt werden können. Die WO 90/11270 beschreibt ein Verfahren zur Entfluorierung von Carbonsäuren mittels Zeolithen, welche eine Kristallinität von mindestens 90 % aufweisen sollen.

Die vorstehenden Ausführungen erläutern die Probleme bei fluororganischen Verbindungen. Auch bei Verbindungen, die nicht mittels Fluor substituiert sind, aber bei deren Herstellung Fluorid eingeschleppt wurde, kann es zu solchen Problemen kommen.

Überraschend ist, daß Korrosion oft auf hydrolysierbares Fluorid zurückzuführen ist.

Aufgabe der vorliegenden Erfindung ist es, ein technisch einfach durchführbares Verfahren anzugeben, mit welchem sich organische Verbindungen, insbesondere fluororganische, reinigen oder herstellen lassen, die verringerte Mengen an hydrolysierbarem Fluorid aufweisen. Besondere Aufgabe ist es, ein Verfahren anzugeben, mit welchem sich Carbonsäuren, Carbonsäurechloride und ihre Derivate, insbesondere aus Kondensationsreaktionen, wie Carbonsäureester in hoher Ausbeute reinigen lassen, die fluoridarm sind bzw. verringerte Mengen an Carbonsäurefluoriden und freier HF aufweisen, bzw. herstellen lassen, wenn man von entsprechend verunreinigten Carbonsäuren, Carbonsäurechloriden oder deren Derivate wie Estern ausgeht. Diese Aufgabe wird durch das Verfahren der vorliegenden Erfindung gelöst.

Das erfindungsgemäße Verfahren zur Herstellung von organischen Verbindungen, die arm an hydrolysierbarem Fluorid sind, sieht vor, die mit hydrolysierbarem Fluorid verunreinigten fluororganischen Verbindungen mit amorphem SiO₂ als Abtrennmittel für hydrolysierbares Fluorid zu kontaktieren.

Das erfindungsgemäße Verfahren eignet sich für die Reinigung organischer Verbindungen, die durch Fluorid oder hydrolysierbares Fluorid verunreinigt sind. Dabei kann diese verunreinigung herstellungsbedingt und/oder durch Hydrolyse von Fluor im Molekül resultieren. Bevorzugt reinigt man fluororganische Verbindungen. Das sind Verbindungen, die mindestens ein Fluoratom aufweisen. Gegebenenfalls können auch noch andere Halogensubstituenten enthalten sein. Anhand dieser Ausführungsform wird die Erfindung weiter erläutert.

Die Erfindung umfaßt zwei Aspekte: die Reinigung bereits hergestellter fluororganischer Verbindungen (z. B. vor ihrer Anwendung oder Weiterverarbeitung oder bei der Lagerung) und die Herstellung von fluororganischen Verbindungen unter gleichzeitiger Reinigung gemäß der Erfindung.

Bevorzugt wendet man es auf die Herstellung oder Reinigung von fluorhaltigen Carbonsäuren, fluorhaltigen Carbonsäurechloriden an sowie auf die Herstellung oder Reinigung von Derivaten von fluorhaltigen Carbonsäuren und fluorhaltigen Carbonsäurechloriden. Derivatisierung bedeutet bevorzugt Kondensationsreaktionen wie Veresterung von fluorhaltigen Carbonsäuren, Carbonsäurechloriden, Kondensation mit Hydrazinderivaten, Hydrolyse etc.

Es kann auch sehr gut auf organische Verbindungen mit einer oder mehreren CF₃-, CF₂H- oder CF₂Cl-Gruppen angewendet werden.

Unter "hydrolysierbarem Fluorid" wird auch Alkalifluorid verstanden, wie es z. B. bei alkalisch (Alkalilauge) katalysierten Reaktionen anfallen kann.

Das erfindungsgemäße Verfahren dient bevorzugt zur Herstellung oder Reinigung von Carbonsäuren, Carbonsäurechloriden und Carbonsäureestern, die arm an Carbonsäurefluorid, freier HF bzw. hydrolysierbarem Fluorid sind, aus Carbonsäuren, Carbonsäurechloriden und Carbonsäureestern, die mit Carbonsäurefluorid, HF bzw. hydrolysierbarem Fluorid verunreinigt sind, unter Kontaktieren derselben mit amorphem SiO₂ als Abtrennmittel für Carbonsäurefluorid, freie HF und hydrolysierbares Fluorid.

Das amorphe Siliciumdioxid liegt beispielsweise gefällt als Hydrat oder in Form von Kieselgel-Perlen vor. Wie schon gesagt, wird bei Kondensationsreaktionen oft Wasser freigesetzt. Dies führt zur HF-Bildung, dies wiederum zur Korrosion. Bei der Reinigung von Substanzen oder Reaktionsgemischen, die außer diesem Kondensationswasser keine weiteren Mengen an Wasser enthalten, ist fein zerteiltes SiO₂, gegebenenfalls als Hydrat, als HF-Fänger gut brauchbar. In Systemen, in denen eine größere Menge Wasser enthalten ist, setzt man zweckmäßig Kieselgel-Perlen (oder anderes Material gröberer Körnung) ein. Man kann die Perlen oder Granulat oder Preßlinge direkt im Reaktor einsetzen. Vorteilhaft führt man das zu reinigende Material bzw. die Reaktionsmischung im Kreislauf über ein separat angeordnetes Bett des partikelförmigen Sorbens. Es wurde beobachtet, daß die HF-bedingte Korrosion auch bei Systemen mit größerem Wassergehalt gut unterdrückt wird (z. B. die Hexafluorokieselsäurebildung oder deren erneute Zersetzung).

Bei zusätzlicher Verwendung von "Onium"-Salzen frischt man diesen HF-Fänger auf, sobald das molare Verhältnis von HF zu "Onium"-Salz den Wert von 2:1 überschritten hat.

Das erfindungsgemäße Verfahren eignet sich besonders zur Herstellung (Reinigung) von Carbonsäuren der Formel R¹C(O)OH, von Carbonsäurechloriden der Formel (I) R¹C(O)Cl und Carbonsäurechloriden der Formel (II) R¹C(O)CH₂C(O)Cl, und deren durch Kondensation erhaltenen Derivaten, wie Estern der Formel (III) R¹C(O)OR² und von Estern der Formel (IV) R¹C(O)CH₂C(O)OR²; die Bedeutung für R¹ und R² sind weiter unten erläutert. Gewünschtenfalls kann man die Reinigung auch durch gleichzeitigen Einsatz von amorphem SiO₂ und eines "Onium"-Salzes oder durch sukzessive Reinigung mit "Onium"-Salz und amorphem SiO₂ in beliebiger Reihenfolge durchführen.

Den Ester kann man nach seiner Herstellung und gegebenenfalls Isolation durch das Erfindungsgemäße Verfahren reinigen. Gemäß einer Ausführungsform nimmt man die Abtrennung von Carbonsäurefluoriden, HF und hydrolysierbarem Fluorid während der Esterherstellung aus Carbonsäurechlorid und Alkohol vor.

Diese Ausführungsform zur Herstellung von fluoridarmen Carbonsäureestern aus Alkoholen und mit Carbonsäurefluorid bzw. HF und hydrolysierbarem Fluorid verunreinigten Carbonsäurechloriden ist dadurch gekennzeichnet, daß man die Umsetzung wasserfrei in Anwesenheit von amorphem SiO₂ oder in Anwesenheit eines "Onium"-Salzes der dem eingesetzten Carbonsäurechlorid entsprechenden Carbonsäure als Abtrennmittel und des amorphem SiO₂ durchführt. Der gebildete Carbonsäureester kann dann z. B. destillativ abgetrennt werden. Das Fluorid verbleibt im Rückstand.

Dabei kann das "Onium"-Salz der Carbonsäure gleichzeitig als Katalysator dienen. Eine Möglichkeit besteht darin, die Umsetzung von Säurechlorid und Alkohol in Anwesenheit des "O-nium"-Salzes durchzuführen und die Reaktionsmischung im Kreislauf über das Amorphe SiO₂ zu führen. Natürlich kann man auch ohne Katalysator oder mit anderen Katalysatoren arbeiten und eine Kreislaufführung der Reaktionsmischung über das amorphe SiO₂ vorsehen.

Das "Onium"-Salz kann man gewünschtenfalls aus der Carbonsäure und der dem "Onium"-Kation entsprechenden Base in situ herstellen. Wenn es sich um eine instabile Säure handelt - beispielsweise um eine β-Keto-Carbonsäure mit Neigung zu Decarboxylierung - kann man zunächst eine andere Carbonsäure einsetzen. So kann man beispielsweise anstelle der 4,4,4-Trifluoracetylessigsäure zunächst die Trifluoressigsäure einsetzen, um das "Onium"-Salz zu erhalten. Es wurde festgestellt, daß auf schonende Weise sich dann bei Zugabe der 4,4,4-Trifluoracetessigsäure oder eines Esters das "Onium"-Salz der 4,4,4,-Trifluoracetessigsäure bildet, ohne daß es zur Decarboxylierung kommt.

Ein Zusatz von Säure, z. B. einer Carbonsäure, ist nicht notwendig und erfolgt vorzugsweise nicht.

Das erfindungsgemäße Verfahren kann prinzipiell für die Herstellung und Reinigung beliebiger Carbonsäuren, Säurechloride und Ester von beliebigen Carbonsäuren mit beliebigen Alkoholen angewendet werden. Eine bevorzugte Ausführungsform sieht vor, daß man eine Carbonsäure der Formel R¹C(O)OH oder R¹C(O)CH₂C(O)OH, Carbonsäurechlorid der Formel R¹C(O)Cl (I) oder R¹C(O)CH₂C(O)Cl (II), einen Ester der Formel (III) R¹C(O)OR² oder der Formel (IV) R¹C(O)OH₂C(O)OR² einsetzt, worin R¹ für durch mindestens 1 Halogenatom, insbesondere durch mindestens 1 Fluoratom substituiertes Alkyl mit 1 bis 6 C-Atomen steht und R² die obige Bedeutung hat. Besonders gut eignet sich das Verfahren zur Anwendung auf Verbindungen der Formel R¹C(O)OH, der Formel (I), der Formel (II) (wobei die Verbindungen der Formel (II) z. B. durch Anlagerung der Verbindungen der Formel (I) an Keten erhältlich sind) der Formeln (III) oder (IV), in welchen R¹ für polyfluoriertes, perfluoriertes oder polyfluorchloriertes Alkyl mit 1 bis 6 C-Atomen, insbesondere 1 bis 4 C-Atomen steht. Dabei bedeutet der Begriff "polyfluoriert", daß mindestens 2/3 aller H-Atome in R¹ gegen F-Atome ausgetauscht sind. Der Begriff "perfluoriert" bedeutet, daß alle H-Atome in R¹ gegen F-Atome ausgetauscht sind. Der Begriff "polyfluorchloriert" bedeutet, daß mindestens 2/3 aller H-Atome in R¹ gegen F-Atome ausgetauscht sind und von den verbleibenden H-Atomen mindestens der überwiegende Teil oder alle gegen Cl-Atome ausgetauscht sind.

Weiterhin ist es bevorzugt, daß man einen Ester oder Alkohol der Formel R²OH (II) einsetzt, worin R² für Alkyl oder Alkenyl mit 1 bis 8 C-Atomen; durch mindestens 1 Halogenatom substituiertes Alkyl oder Alkenyl mit 1 (bzw. im Falle des Alkenyls mit mindestens 2 C-Atomen) bis 8 C-Atomen; Phenyl, Tolyl; Benzyl; durch mindestens 1 Halogenatom und/oder mindestens eine Nitrogruppe substituiertes Phenyl, Tolyl oder Benzyl steht.

Ganz besonders bevorzugt ist es, daß R¹ für Polyfluoralkyl, Perfluoralkyl oder Polyfluorchloralkyl mit 1 bis 4 C-Atomen und R² für Alkyl oder Alkenyl mit 1 (bzw. im Falle des Alkenyls mit mindestens 2 C-Atomen) bis 4 C-Atomen; durch mindestens 1 Halogenatom substituiertes Alkyl oder Alkenyl mit 1 (bzw. im Falle des Alkenyls mit mindestens 2 C-Atomen) bis 4 C-Atomen; Phenyl; durch mindestens 1 Halogenatom und/oder durch mindestens eine Nitrogruppe substituiertes Phenyl, steht. Insbesondere bedeutet R¹ Perfluormethyl, Perfluorethyl, Perfluorpropyl oder Chlordifluormethyl. Insbesondere bevorzugt steht R² für Alkyl oder Alkenyl mit 1 (bzw. im Falle des Alkenyls mit mindestens 2 C-Atomen) bis 3 C-Atomen; durch mindestens 1 Fluoratom substituiertes Alkyl oder Alkenyl mit 1 (bzw. im Falle des Alkenyls mit mindestens 2 C-Atomen) bis 3 C-Atomen; Phenyl; durch mindestens 1 Fluoratom und/oder mindestens eine Nitrogruppe substituiertes Phenyl.

Durch Fluor und gegebenenfalls Chlor substituierte Carbonsäurechloride können auf bekannte Weise hergestellt werden.

Der Begriff "Onium" steht für Kationen mit positiv geladenem Stickstoff, beispielsweise protonierte aromatische Stickstoffbasen wie Pyridinium oder protonierte Alkyl-, Dialkyl- oder Trialkylammonium-Kationen mit bis zu 20 C-Atomen, oder für durch Cycloalkyl substituierte Ammonium-Verbindungen oder cycloaliphatische Stickstoffbasen wie Piperidinium oder quartäre Ammoniumkationen.

Sehr gut geeignet als Carbonsäuresalz sind "Onium"-Salze, wobei "Onium" für ein Kation des Stickstoffs der Formel R^{I}R^{II}R^{III}R^{IV}N⁺, worin R^{I}, R^{II}, R^{III} und R^{IV} unabhängig voneinander Wasserstoff, Alkyl mit 1 bis 20 C-Atomen, Aryl oder Aralkyl stehen, oder worin R^{I} und R^{II} oder worin R^{III} und R^{IV}, oder worin R^{I}, R^{II} und R^{III} oder worin R^{I}, R^{II}, R^{III} und R^{IV}, gegebenenfalls unter Einschluß des Stickstoffatoms, gesättigte oder ungesättigte Ringsysteme bilden. "Aryl" bedeutet hier insbesondere Phenyl oder durch 1 oder mehrere C1-C2-Alkylgruppen substituiertes Phenyl. Hervorragend geeignet sind Salze, in denen "Onium" für Ammonium, Pyridinium oder R^{1'} R^{2'} R^{3'} R^{4'} N⁺ steht, worin R^{1'}, R^{2'}, R^{3'} und R^{4'} unabhängig voneinander Wasserstoff, Alkyl mit 1 bis 15 C-Atomen, Phenyl oder Benzyl stehen. Als Beispiel für solche Kationen seien genannt Pyridinium, Piperidinium, Anilinium, Benzyltriethylammonium und Triethylammonium.

Besonders gut geeignet ist das erfindungsgemäße Verfahren zur Herstellung bzw. Reinigung der Difluoressigsäure, 4,4-Difluoracetessigsäure, Trifluoressigsäure, Chlordifluoressigsäure, 4,4,4-Trifluoracetessigsäure und 4-Chlor-4,4-difluoracetessigsäure, ihren Säurechloriden und ihren durch Kondensationsreaktionen (Hydrolyse, Veresterung, Hydrazinolyse unter Bildung von Ringen mit Heteroatomen) erhaltenen Derivaten wie den Estern mit 1.1.1-Trifluorethanol, Methanol, Ethanol, Isopropanol, n-Propanol, 4-Nitro-Phenol, Pentafluorphenol und Allylalkohol.

Bei der Esterherstellung liegt das Molverhältnis zwischen Carbonsäurehalogenid und Alkohol vorteilhafterweise oberhalb von 0,9. Der Alkohol kann auch in höherem Überschuß eingesetzt werden und dient als Lösungsmittel, besonders wenn es sich um einen durch elektronenziehende Gruppen, beispielsweise Fluor-Atome, substituierten Alkohol handelt. Zweckmäßig liegt das Molverhältnis zwischen Alkohol und Carbonsäurehalogenid zwischen 0,9:1 und 1,1:1, bzw. falls der Alkohol als Lösungsmittel dient, bis hin zu 5:1.

Die Temperatur, bei der die Umsetzung (bzw. die Reinigung) durchgeführt wird, liegt bei Umgebungstemperatur (etwa 20 °C) bis hin zum Siedepunkt der Mischung, beispielsweise bis hin zu 100 °C. Bei instabilen Säuren oder Säurechloriden arbeitet man unterhalb der Decarboxylierungstemperatur. Dies gilt z. B. bei der Veresterung des 4,4,4-Trifluor-, 4-Chlor-4,4-difluor- und 4,4-Difluoracetessigsäurechlorides; hier verestert man bei Raumtemperatur oder unter Kühlung der Reaktionsmischung. Man arbeitet bei Umgebungsdruck (etwa 1 bar abs.) oder gewünschtenfalls auch bei erhöhtem Druck, beispielsweise bei einem Druck von bis zu 5 bar abs.

Das "Onium"-Salz kann in katalytischen oder molaren Mengen anwesend sein. Zweckmäßig liegt das Molverhältnis zwischen Säurehalogenid und dem Carbonsäuresalz im Bereich von 1:1 bis 20000:1.

Neben der schon erwähnten Destillation zur Isolierung der Ester kann man bei einigen Estern den Umstand nutzen, daß sich zwei Phasen bilden: eine Phase enthält den sehr reinen Ester (>94 % Reinheit), die andere Phase den Katalysator, den Alkohol und das Fluorid. Zwei Phasen bilden sich z. B. bei den Methyl- und Ethylestern der Trifluor- und Chlordifluoressigsäure sowie den n-Propylester der Chlordifluoressigsäure. Dies hat den Vorteil der vereinfachten Aufarbeitung.

Diese Ausführungsform des erfindungsgemäßen Verfahrens zur Herstellung von Methyl- oder Ethylestern der Trifluoressigsäure und der Chlordifluoressigsäure und des n-Propylesters der Chlordifluoressigsäure sieht vor, daß man das Säurechlorid mit einem Überschuß des Alkohols in Anwesenheit eines "Onium"-Salzes der betreffenden Säure umsetzt und das Molverhältnis von Alkohol zu Säurechlorid so wählt, daß sich zwei Phasen bilden, wobei eine Phase den Ester in einer, ohne Destillationsstufe erzielbaren Reinheit von mindestens 95 Gew.-% enthält, und man den Ester durch Abtrennen der Esterphase von der anderen Phase isoliert. Bei dieser Vorgehensweise fällt der Ester somit in einer Reinheit an, die eine Destillation erübrigt. Eine bevorzugte Ausführungsform des erfindungsgemäßen Verfahrens sieht deshalb die Isolierung des erhaltenen Esters ohne Destillation vor.

Bei der Herstellung des Methylesters der Trifluoressigsäure liegt das Molverhältnis von Methanol zu Trifluoracetylchlorid im Bereich von 1,03:1 bis 4:1. Bei der Herstellung des Ethylesters der Trifluoressigsäure liegt das Molverhältnis von Ethanol zu Trifluoracetylchlorid im Bereich von 1,01:1 bis 5:1. Bei der Herstellung des Methylesters der Chlordifluoressigsäure liegt das Molverhältnis von Methanol zu Chlordifluoracetylchlorid im Bereich von 1,06:1 bis 2,5:1. Bei der Herstellung des Ethylesters der Chlordifluoressigsäure liegt das Molverhältnis von Ethanol zu Chlordifluoracetylchlorid im Bereich von 1,02:1 bis 2,5:1. In den genannten Bereichen liegen zwei Phasen vor, in welchen wie gesagt eine Phase den Ester umfaßt, der stets in einer Reinheit von mindestens 95 Gew.-% enthalten ist. Die Methylester bilden stets die untere Phase; der Ethylester der Chlordifluoressigsäure bildet ebenfalls die untere Phase, der Ethylester der Trifluoressigsäure bildet die obere Phase.

Die Erfindung liefert Säuren, Säurechloride und Ester mit stark verringertem Fluoridgehalt (z. B. unter 70 ppm, ja sogar von 10 ppm und weniger) je nach ursprünglichem Gehalt an HF, Carbonsäurefluorid und hydrolysierbarem Fluorid. Einerseits ist das Produkt somit sehr rein, andererseits ergeben sich keine Korrosionsprobleme (oder stark verringerte Korrosion) z. B. bei der Veresterung in Anlagen und Anlagenbauteilen aus Keramik oder Glas.

Die Verwendung von "Onium"-Salzen als Katalysator bei der Veresterung wurde bereits in der EP-A 623 582 (= US 5,405,991) offenbart. Daß auf diese Weise nicht nur die Herstellung sehr einfach möglich ist, sondern auch bei Einsatz carbonsäurefluoridhaltiger Ausgangsstoffe fluoridarmes Produkt erhalten wird und die Korrosion verringert wird, geht aus dieser Anmeldung nicht hervor. Gleiches gilt für die nicht vorveröffentlichte DE 197 32 031, die die 2-Phasen-Methode zur Herstellung der Methyl- und Ethylester von CF₃C(O)Cl und CF₂ClC(O)Cl betrifft.

"Onium"-Salze der 4,4,4-Trifluoracetessigsäure, der 4-Chlor-4,4-difluoracetessigsäure und der 4,4-Difluoracetessigsäure sowie die freien Säuren selbst sind brauchbar für das erfindungsgemäße Verfahren.

Die Erfindung wird anhand der folgenden Beispiele weiter erläutert, ohne daß sie in ihrem Umfang eingeschränkt werden soll. Nur die Beispiele 5, 6 und 8-10 sind als erfindungsgemäß enzusehen.

### Allgemeine Versuchsvorschrift für Beispiele 1 - 3 zur Herstellung von fluoridarmen Trifluoressigsäureethylester aus Trifluoracetylchlorid und Ethanol mit Katalysator

| Ansatz (gilt für Beispiele 1 - 3): | |
|---|---|
| 0,2 mol Pyridin | 15,8 g |
| 0,2 mol Trifluoressigsäure (TFA) | 22,8 g |
| 2,0 mol Ethanol p.A. | 92,1 g |
| 1,8 mol Trifluoracetylchlorid (TFAC) | 238,5 g |

### Durchführung:

In einem 250 ml Dreihalskolben mit Magnetrührstab, Thermofühler und Trockeneiskühler wurde das Pyridin vorgelegt und TFA zugetropft. Die Reaktion ist exotherm, und bevor das Salz komplett ausfallen kann, wurde das Ethanol zugegeben, um es in Lösung zu halten. Um die Reaktionsgeschwindigkeit zu erhöhen, wurde die Lösung im Ölbad auf 50 °C temperiert und bei dieser Temperatur wird das TFAC über eine Glasfritte eingeleitet.

Bei 20 % des benötigten TFAC's bildeten sich zwei Phasen, wobei die obere Phase fast reiner Trifluoressigsäuereethylester ist.

Die Lösung wurde nach Beendigung des Einleitens noch eine halbe Stunde nachgerührt, danach in einen Scheidetrichter überführt. Beide Phasen sind nach der Trennung klar, die Katalysatorphase ist leicht gelb gefärbt.

### Beispiel 1:

Hierbei wurde TFAC mit einem Fluoridgehalt von 570 ppm verwendet. Die Versuchsdurchführung erfolgte, wie oben beschrieben, und ergab nach der Reaktion eine Esterphase mit einem Fluoridgehalt von 61 ppm und eine Katalysatorphase mit 1850 ppm F-.

Die prozentuale Verteilung zeigte, daß sich das Fluorid bevorzugt in der Katalysatorphase befindet; dort wurden 86,03 % des Gesamtfluorides gefunden, die Esterphase enthielt noch 15,27 %.

### Beispiel 2:

Hierbei wurde das gleiche TFAC mit einem Fluoridgehalt von 570 ppm verwendet. Die Versuchsdurchführung erfolgte, wie oben beschrieben, allerdings mit deutlich stärkerer Rührung und ergab nach der Reaktion eine Esterphase mit einem Fluoridgehalt von 10 ppm und eine Katalysatorphase mit 3670 ppm F-.

Die prozentuale Verteilung zeigte, daß sich das Fluorid bevorzugt in der Katalysatorphase befindet; dort wurden 97,28 % des Gesamtfluorides gefunden; in der Esterphase war der Fluoridgehalt auf 2,72 % des ursprünglichen Wertes verringert worden.

### Beispiel 3:

Hierbei wurde TFAC mit einem Fluoridgehalt von 71 ppm verwendet. Die Versuchsdurchführung erfolgte, wie oben beschrieben, und ergab nach der Reaktion eine Esterphase mit einem Fluoridgehalt von 10 ppm und eine Katalysatorphase mit 130 ppm F-.

Die prozentuale Verteilung zeigte, daß sich das Fluorid bevorzugt in der Katalysatorphase befindet; dort wurden 76,66 % des Gesamtfluorides gefunden, die Esterphase enthielt noch 23,35 % des ursprünglichen Wertes.

Die Beispiele zeigen, daß der Fluoridwert im Ester auf sehr geringe Werte herabgesetzt werden kann, sowohl bei ursprünglich recht hohem als auch bei ursprünglich schon recht niedrigem Fluoridgehalt im Carbonsäurefluorid.

### Beispiel 4:

| Ansatz: | |
|---|---|
| 0,1 mol Pyridin | 7,9 g |
| 0,1 mol Trifluoressigsäure | 11,4 g |
| 2,0 mol Ethanol p.A. | 92,1 g |
| 1,8 mol Trifluoracetylchlorid | 238,5 g |

Hierbei wurde wieder TFAC mit einem Fluoridgehalt von 570 ppm verwendet. Die Katalysatormenge wurde auf 5 mol %, statt der sonst verwendeten 10 mol % reduziert. Die Versuchsdurchführung erfolgte, wie oben beschrieben, nur mit deutlich stärkerer Rührung und ergab nach der Reaktion eine Esterphase mit einem Fluoridgehalt von 32 ppm.

### Beispiel 5:

Herstellung von fluoridarmem Trifluoressigsäureester unter Verwendung von SiO₂ in einem Keramik-Rührkessel
5.1. Eine Lösung von 0,10 kg Pyridiniumtrifluoracetat in 1,90 kg Methanol wurde hergestellt und mit weiteren 4,80 kg Methanol vermischt. 0,02 kg gefälltes SiO₂-Hydrat (Produkt "1.00656.000 Kieselsäure gefällt reinst schwer" der Firma Merck KGaA, Darmstadt; Schüttgewicht etwa 30 - 50 g/100 ml), Korngröße <0,1 mm, wurde zugegeben und unter Rühren 19,2 kg Trifluoracetylchlorid (1000 ppm hydrolysierbares Fluorid) eingeleitet. Der Methylester enthielt nach der Destillation weniger als 40 ppm hydrolysierbares Fluorid. Auch nach vielfacher Wiederholung war an den Keramikteilen des Rührkessels keine Korrosion zu erkennen.
5.2 Beispiel 5.1 wurde wiederholt. Statt Methanol wurde die gleiche Mol-Menge an Ethanol eingesetzt. Der Ethylester enthielt nach der Destillation weniger als 30 ppm hydrolysierbares Fluorid.

### Beispiel 6:

### Abtrennung von hydrolysierbarem Fluorid aus Trifluoracetylchlorid

In einem Glasrohr mit 1,5 cm Innendurchmesser wurde eine Schüttung von 100 g KC-Trockenperlen AF 125 der Engelhard Process Chemicals GmbH, Hannover, erzeugt. Diese Trockenperlen bestehen aus SiO₂-Gel und haben einen Durchmesser zwischen 2 und 5 mm. Der Porendurchmesser beträgt 125 Å (12,5 nm). Sie werden üblicherweise als Trockenmittel eingesetzt oder als Katalysatorträger.

Über diese Schüttung wurde bei Raumtemperatur Trifluoracetylchlorid mit 570 ppm hydrolysierbarem Fluorid geleitet. Das die Schüttung verlassende Produkt wies einen Gehalt von 98 ppm auf.

### Beispiele 7 - 9:

### Reinigung von 4,4,4-Trifluoracetessigsäureethylester

630 g des Esters (3,4 mol) wurden mit 2,6 g HF (0,1 mol) versetzt, um einen mit 4125 ppm hydrolysierbarem Fluorid verunreinigten Ester zu simulieren.

### Beispiel 7:

### Py.TFA als F-Abtrennmittel

Hierbei wurde in einem 250 ml Teflonkolben mit Magnetrührstab 2,1 g Pyridin (0,027 mol) vorgelegt und mit 3,1 g Trifluoressigsäure (0,027 mol) (die 4,4,4-Trifluoracetessigsäure neigt zum Decarboxylieren bei direkter Herstellung des Salzes aus Pyridin und der 4,4,4-Trifluoracetessigsäure) versetzt. Zu dem Pyridiniumtrifluoracetat wurde nun 50,2 g 4,4,4-Trifluoracetessigsäureethylester gegeben und 3 Stunden bei Raumtemperatur gerührt. Nach dieser Zeit wurde die Lösung noch 1 Stunde bei 70 °C Wasserbadtemperatur erhitzt und unter Vakuum abdestilliert. Die Sumpfprobe, in welcher das "Onium"-Salz der 4,4,4-Trifluoracetessigsäure nachgewiesen (¹⁹F-NMR) wurde, ergab einen Fluoridwert von 14500 ppm. Der abdestillierte Ester enthielt noch 1460 ppm Fluorid.

Analog können auch die "Onium"-Salze der 4,4-Difluoracetessigsäure und der 4-Chlor-4,4-difluoracetessigsäure erzeugt werden. Die Isolierung ist nach Standard-Methoden möglich.

### Beispiel 8:

### Gefälltes SiO₂-Hydrat als Sorbens

Hierbei wurde 50,1 g des 4,4,4-Trifluoracetessigsäureethylester (0,27 mol) mit Magnetrührstab in einen Teflonkolben gegeben. Zu dem 4,4,4,-Trifluoracetessigsäureethylester wurde noch 6,57 g gefälltes SiO₂-Hydrat der Firma Merck (siehe Beispiel 5) gegeben und 3 Stunden bei Raumtemperatur gerührt. Nach dieser Zeit wird die Lösung noch 1 Stunde bei 80 °C Wasserbadtemperatur erhitzt. Die Lösung wird nach Beendigung der Rührzeit warm abfiltriert und die Lösung auf Fluorid untersucht. Wir erhielten einen Fluoridgehalt von 9 ppm.

### Beispiel 9:

### SiO₂-Gelperlen als Sorbens

Hierbei wurde 50,7 g des 4,4,4-Trifluoracetessigsäureethylester (0,28 mol) mit Magnetrührstab in einen Teflonkolben gegeben. Zu dem 4,4,4-Trifluoracetessigsäureethylester wurde noch 10,2 g Trockenperlen "AF 125" gegeben (näheres siehe Beispiel 6), und 3 Stunden bei Raumtemperatur gerührt. Nach dieser Zeit wurde 4,4,4,-Trifluoracetessigsäureethylester abfiltriert, Fluoridgehalt: 47 ppm.

### Beispiel 10:

### Reinigung von Trifluoressigsäure

Trifluoracetylchlorid mit ca. 1000 ppm hydrolysierbarem Fluorid wurde mit der nahezu äquimolaren Menge Wasser gerührt. Kontinuierlich wurde die Reaktionsmischung über "AF 125" im Kreislauf geführt. Im Produkt wurden weniger als 50 ppm hydrolysierbares Fluorid nachgewiesen. Somit sorbiert das SiO₂ das Fluorid trotz des Wassergehalts der Reaktionsmischung.

### Beispiel 11:

### Herstellung und Isolation von 4,4,4-Trifluoracetessigsäure

| Ansatz: | | |
|---|---|---|
| 4,0 mol | α,α,α Trifluoracetessigsäureethylester | 736,4 g |
| 2,0 mol | Trifluoressigsäure | 228,0 g |
| 0,9 mol | Schwefelsäure 95-97% | 90,0 g |

### Aufbau u. Durchführung:

In einem 1-Liter-Kolben mit Destillationsaufsatz wurden α,α,α-Trifluoracetessigsäureethylester und Trifluoressigsäure vorgelegt, dazu wurde vorsichtig konzentrierte Schwefelsäure getropft. Die zuvor klare Lösung trübte sich dabei ein. Danach wurde 1,5 Stunden bei 70 °C - 90 °C gekocht. Bei Beobachten von vereinzelt auftretenden Gasblasen am Blasenzähler wurde die Temperatur etwas zurückgenommen.

Nach dem Kochen wurde die hellbraune Lösung, aus dem Ölbad genommen und zum Abkühlen in ein Eisbad gestellt, wo sich nach kurzer Zeit feine, weiße, nadelförmige Kristalle ausbildeten. Die Kristalle wurden über eine Glasfritte abgesaugt und mittels NMR und Massenspektrometrie analysiert und als Trifluoracetessigsäure bestätigt.

4,4-Difluoracetessigsäure und 4-Chlor-4,4-difluoracetessigsäure können in analoger Weise aus dem Ethylester und Trifluoressigsäure und anschließenden üblichen Reinigungsoperationen gewonnen werden.

## Patentansprüche

1. Verfahren zur Herstellung oder Reinigung von fluorierten organischen Verbindungen mit verringerten Mengen an hydrolysierbarem Fluorid, wobei man die mit hydrolysierbarem Fluorid verunreinigten organischen Verbindungen mit amorphem SiO₂ als Abtrennmittel für hydrolysierbares Fluorid kontaktiert.

2. Verfahren nach Anspruch 1, **dadurch gekennzeichnet, daß** man Carbonsäuren oder Carbonsäurechloride reinigt.

3. Verfahren nach Anspruch 1, **dadurch gekennzeichnet, daß** man Derivate von Carbonsäuren oder Carbonsäurechloriden reinigt.

4. Verfahren nach einem der Ansprüche 1 bis 3, **dadurch gekennzeichnet, daß** man die Derivate von Carbonsäuren oder Carbonsäurechloriden bei ihrer Herstellung reinigt.

5. Verfahren nach Anspruch 1 zur Herstellung von Carbonsäuren, Carbonsäurechloriden und Carbonsäureestern, die arm an Carbonsäurefluorid bzw. hydrolysierbarem Fluorid sind, aus mit Carbonsäurefluorid bzw. hydrolysierbarem Fluorid verunreinigten Carbonsäuren, Carbonsäurechloriden und Carbonsäureestern, unter Kontaktieren derselben mit amorphem SiO₂ als Abtrennmittel für Carbonsäurefluorid und hydrolysierbares Fluorid.

6. Verfahren nach Anspruch 1, **dadurch gekennzeichnet, daß** man Kieselgelperlen oder gefälltes SiO₂-Hydrat verwendet.

7. Verfahren nach Anspruch 5, **dadurch gekennzeichnet, daß** man Carbonsäureester in Anwesenheit von amorphem SiO₂ oder in Anwesenheit von amorphem SiO₂ und in Anwesenheit oder in Anwesenheit von amorphem SiO₂ und in Anwesenheit eines "Onium"-Salzes der entsprechenden Carbonsäure aus Carbonsäurechlorid und Alkohol herstellt.

8. Verfahren nach einem der vorhergehenden Ansprüche, **dadurch gekennzeichnet, daß** man eine Carbonsäure der Formel R¹C(O)OH reinigt oder ein Carbonsäurechlorid der Formel R¹C(O)Cl (I) oder R¹C(O)CH₂C(O)Cl (II) einsetzt, worin R¹ für durch mindestens 1 Halogenatom substituiertes Alkyl mit 1 bis 6 C-Atomen; insbesondere Polyfluoralkyl, Perfluoralkyl oder Polyfluorchloralkyl mit 1 bis 6 C-Atomen steht.

9. Verfahren nach Anspruch 7, **dadurch gekennzeichnet, daß** man einen Alkohol der Formel R²OH (II) einsetzt, worin R² für Alkyl oder Alkenyl mit 1 bis 8 C-Atomen; durch mindestens 1 Halogenatom substituiertes Alkyl oder Alkenyl mit 1 bis 8 C-Atomen; Phenyl, Tolyl; Benzyl; durch mindestens 1 Halogenatom und/oder mindestens eine Nitrogruppe substituiertes Phenyl, Tolyl oder Benzyl steht.

10. Verfahren nach Anspruch 5, **dadurch gekennzeichnet, daß** man einen Ester der Formel (III) R¹C(O)OR² oder (IV) R¹C(O)CH₂C(O)OR² reinigt oder herstellt, wobei R¹ und R² die vorstehende Bedeutung besitzen.

11. Verfahren nach einem der Ansprüche 8 bis 10, **dadurch gekennzeichnet, daß** R¹ für Polyfluoralkyl, Perfluoralkyl oder Polyfluorchloralkyl mit 1 bis 4 C-Atomen und R² für Alkyl oder Alkenyl mit 1 bis 4 C-Atomen; durch mindestens 1 Halogenatom substituiertes Alkyl oder Alkenyl mit 1 bis 4 C-Atomen; Phenyl; durch mindestens 1 Halogenatom und/oder durch mindestens eine Nitrogruppe substituiertes Phenyl steht.

12. Verfahren nach Anspruch 11, **dadurch gekennzeichnet, daß** R¹ für Perfluormethyl, Perfluorethyl, Perfluorpropyl oder Chlordifluormethyl steht.

13. Verfahren nach Anspruch 12, **dadurch gekennzeichnet, daß** R² für Alkyl oder Alkenyl mit 1 bis 3 C-Atomen; durch mindestens 1 Fluoratom substituiertes Alkyl oder Alkenyl mit 1 bis 3 C-Atomen; Phenyl; durch mindestens 1 Fluoratom und/oder mindestens eine Nitrogruppe substituiertes Phenyl steht.

14. Verfahren nach Anspruch 7, **dadurch gekennzeichnet, daß** das Mol-Verhältnis zwischen Säurechlorid und "Onium"-Salz im Bereich von 1:1 bis 20000:1 liegt.

15. Verfahren nach Anspruch 14, **dadurch gekennzeichnet, daß** man ein Pyridinium- oder Piperidinium-Salz einsetzt.

16. Verfahren nach Anspruch 7, wobei man das "Onium"-Salz in situ aus der entsprechenden Carbonsäure und der dem Onium-Kation entsprechenden Base in situ herstellt.

17. Verfahren nach Anspruch 16, wobei man bei zur Decarboxylierung neigenden Carbonsäuren von einer gegenüber Decarboxylierung stabilen Carbonsäure ausgeht.

18. Verfahren nach Anspruch 12, **dadurch gekennzeichnet, daß** man R¹C(O)CH₂C(O)Cl einsetzt, welches durch Reaktion von R¹C(O)Cl und Keten erhalten wurde.

19. Verfahren nach Anspruch 1, **dadurch gekennzeichnet, daß** man CF₃C(O)Cl oder CF₃C(O)CH₂C(O)Cl reinigt oder herstellt oder bei der Derivatisierung, insbesondere der Esterherstellung einsetzt.

20. Verfahren nach Anspruch 7 zur Herstellung von Methyloder Ethylestern der Trifluoressigsäure und der Chlordifluoressigsäure, wobei man das Säurechlorid mit einem stöchiometrischen Überschuß des Alkohols in Anwesenheit des "Onium"-Salzes und von amorphem SiO₂ umsetzt und das Molverhältnis von Alkohol zu Säurechlorid so wählt, daß sich zwei Phasen bilden, wobei eine Phase den Ester in einer, ohne Destillationsstufe erzielbaren Reinheit von mindestens 95 Gew.-% enthält, und man den Ester durch Abtrennen der Esterphase von der anderen Phase isoliert.

21. Verfahren nach Anspruch 20, **dadurch gekennzeichnet, daß** man den Methylester der Trifluoressigsäure herstellt und das Molverhältnis von Methanol zu Trifluoracetylchlorid im Bereich von 1,03:1 bis 4:1 beträgt.

22. Verfahren nach Anspruch 20, **dadurch gekennzeichnet, daß** man den Ethylester der Trifluoressigsäure herstellt und das Molverhältnis von Ethanol zu Trifluoracetylchlorid im Bereich von 1,01:1 bis 5:1 liegt.

23. Verfahren nach Anspruch 20, **dadurch gekennzeichnet, daß** man den Methylester von Chlordifluoressigsäure herstellt und das Molverhältnis von Methanol zu Chlordifluoracetylchlorid im Bereich von 1,06:1 bis 2,5:1 liegt.

24. Verfahren nach Anspruch 20, **dadurch gekennzeichnet, daß** man den Ethylester der Chlordifluoressigsäure herstellt und das Molverhältnis von Ethanol zu Chlordifluoracetylchlorid im Bereich von 1,02:1 bis 2,5:1 liegt.

25. Verfahren nach Anspruch 20, **dadurch gekennzeichnet, daß** man die Reaktionsmischung im Kreislauf führt und mit dem Sorptionsmittel kontaktiert.

26. Verfahren nach Anspruch 6, **dadurch gekennzeichnet, daß** man in wasserhaltigen Systemen Kieselgelperlen einsetzt und im Umlaufstrom reinigt.

27. Verfahren nach Anspruch 1, **dadurch gekennzeichnet, daß** das Molverhältnis zwischen "Onium"-Salz und HF bei oder oberhalb 1:2 liegt.

## Claims

1. A process for the preparation or purification of fluorinated organic compounds with reduced amounts of hydrolysable fluoride, wherein the organic compounds contaminated with hydrolysable fluoride are contacted with amorphous SiO₂ as separating agent for hydrolysable fluoride.

2. A process according to Claim 1, **characterised in that** carboxylic acids or carboxylic acid chlorides are purified.

3. A process according to Claim 1, **characterised in that** derivatives of carboxylic acids or carboxylic acid chlorides are purified.

4. A process according to one of Claims 1 to 3, **characterised in that** the derivatives of carboxylic acids or carboxylic acid chlorides are purified upon the preparation thereof.

5. A process according to Claim 1 for the preparation of carboxylic acids, carboxylic acid chlorides and carboxylic acid esters which are low in carboxylic acid fluoride or hydrolysable fluoride, from carboxylic acids, carboxylic acid chlorides and carboxylic acid esters which are contaminated with carboxylic acid fluoride or hydrolysable fluoride, by contacting them with amorphous SiO₂ separating agent for carboxylic acid fluoride and hydrolysable fluoride.

6. A process according to Claim 1, **characterised in that** silica gel beads or precipitated SiO₂ hydrate is used.

7. A process according to Claim 5, **characterised in that** carboxylic acid esters are prepared in the presence of amorphous SiO₂ or in the presence of amorphous SiO₂ and in the presence of an "onium" salt of the corresponding carboxylic acid from carboxylic acid chloride and alcohol.

8. A process according to one of the preceding claims, **characterised in that** a carboxylic acid of the formula R¹C(O)OH is purified or a carboxylic acid chloride of the formula R¹C(O)Cl (I) or R¹C(O)CH₂C(O)Cl (II) is used, wherein R¹ stands for alkyl with 1 to 6 C atoms which is substituted by at least 1 halogen atom; in particular polyfluoroalkyl, perfluoroalkyl or polyfluorochloroalkyl with 1 to 6 C atoms.

9. A process according to Claim 7, **characterised in that** an alcohol of the formula R²OH (II) is used, wherein R² stands for alkyl or alkenyl with 1 to 8 C atoms; alkyl or alkenyl with 1 to 8 C atoms which is substituted by at least 1 halogen atom; phenyl, tolyl; benzyl; or phenyl, tolyl or benzyl substituted by at least 1 halogen atom and/or at least one nitro group.

10. A process according to Claim 5, **characterised in that** an ester of Formula (III) R¹C(O)OR² or (IV) R¹C(O)CH₂C(O)OR² is purified or prepared, wherein R¹ and R² have the above meaning.

11. A process according to one of Claims 8 to 10, **characterised in that** R¹ stands for polyfluoroalkyl, perfluoroalkyl or polyfluorochloroalkyl with 1 to 4 C atoms and R² for alkyl or alkenyl with 1 to 4 C atoms; alkyl or alkenyl with 1 to 4 C atoms which is substituted by at least 1 halogen atom; phenyl; or phenyl substituted by at least 1 halogen atom and/or at least one nitro group.

12. A process according to Claim 11, **characterised in that** R¹ stands for perfluoromethyl, perfluoroethyl, perfluoropropyl or chlorodifluoromethyl.

13. A process according to Claim 12, **characterised in that** R² stands for alkyl or alkenyl with 1 to 3 C atoms; alkyl or alkenyl with 1 to 3 C atoms which is substituted by at least 1 fluorine atom; phenyl; or phenyl substituted by at least 1 fluorine atom and/or at least one nitro group.

14. A process according to Claim 7, **characterised in that** the molar ratio between acid chloride and "onium" salt lies in the range from 1:1 to 20,000:1.

15. A process according to Claim 14, **characterised in that** a pyridinium or piperidinium salt is used.

16. A process according to Claim 7, wherein the "onium" salt is prepared *in situ* from the corresponding carboxylic acid and the base corresponding to the onium cation.

17. A process according to Claim 16, wherein in the case of carboxylic acids which tend towards decarboxylation the starting point is a carboxylic acid which is stable with respect to decarboxylation.

18. A process according to Claim 12, **characterised in that** R¹C(O)CH₂C(O)Cl which has been obtained by reacting R¹C(O)Cl and ketene is used.

19. A process according to Claim 1, **characterised in that** CF₃C(O)Cl or CF₃C(O)CH₂C(O)Cl is purified or prepared or is used in the derivatisation, in particular the ester preparation.

20. A process according to Claim 7 for the preparation of methyl or ethyl esters of trifluoroacetic acid and chlorodifluoroacetic acid, wherein the acid chloride is reacted with a stoichiometric excess of the alcohol in the presence of the "onium" salt and amorphous SiO₂ and the molar ratio of alcohol to acid chloride is selected such that two phases form, wherein one phase contains the ester in a purity which can be achieved without a distillation stage, of at least 95% by weight, and the ester.is isolated by separating off the ester phase from the other phase.

21. A process according to Claim 20, **characterised in that** the methyl ester of trifluoroacetic acid is prepared and the molar ratio of methanol to trifluoroacetyl chloride is in the range from 1.03:1 to 4:1.

22. A process according to Claim 20, **characterised in that** the ethyl ester of trifluoroacetic acid is prepared and the molar ratio of ethanol to trifluoroacetyl chloride is in the range from 1.01:1 to 5:1.

23. A process according to Claim 20, **characterised in that** the methyl ester of chlorodifluoroacetic acid is prepared and the molar ratio of methanol to chlorodifluoroacetyl chloride is in the range from 1.06:1 to 2.5:1.

24. A process according to Claim 20, **characterised in that** the ethyl ester of chlorodifluoroacetic acid is prepared and the molar ratio of ethanol to chlorodifluoroacetyl chloride is in the range from 1.02:1 to 2.5:1.

25. A process according to Claim 20, **characterised in that** the reaction mixture is circulated and is contacted with the sorbent.

26. A process according to Claim 6, **characterised in that** in water-containing systems silica gel beads are used and purification is effected in a circulating stream.

27. A process according to Claim 1, **characterised in that** the molar ratio of "onium" salt to HF is at or above 1:2.

## Revendications

1. Procédé de préparation ou de purification de composés organiques fluorés comportant de faibles teneurs en fluorure hydrolysable, dans lequel on met en contact les composés organiques contaminés par du fluorure hydrolysable avec SiO₂ amorphe en tant qu'agent de séparation du fluorure hydrolysable.

2. Procédé selon la revendication 1, **caractérisé en ce que** l'on purifie des acides carboxyliques ou des chlorures d'acides carboxyliques.

3. Procédé selon la revendication 1, **caractérisé en ce que** l'on purifie des dérivés d'acides carboxyliques ou de chlorures d'acides carboxyliques.

4. Procédé selon l'une des revendications 1 à 3, **caractérisé en ce que** l'on purifie les dérivés d'acides carboxyliques ou de chlorures d'acides carboxyliques lors de leur préparation.

5. Procédé selon la revendication 1, pour préparer des acides carboxyliques, des chlorures d'acides carboxyliques et des esters d'acides carboxyliques, qui présentent une faible teneur en fluorure d'acide carboxylique ou en fluorure hydrolysable, à partir d'acides carboxyliques, de chlorures d'acides carboxyliques et d'esters d'acides carboxyliques contaminés par du fluorure d'acide carboxylique ou du fluorure hydrolysable, par mise en contact de ceux-ci avec SiO₂ amorphe en tant qu'agent de séparation du fluorure d'acide carboxylique et du fluorure hydrolysable.

6. Procédé selon la revendication 1, **caractérisé en ce que** l'on utilise des perles de gel de silice ou un hydrate de SiO₂ précipité.

7. Procédé selon la revendication 5, **caractérisé en ce que** l'on prépare, à partir de chlorure d'acide carboxylique et d'alcool, un ester d'acide carboxylique en présence de SiO₂ amorphe ou en présence de SiO₂ amorphe et en présence d'un sel "d'onium" de l'acide carboxylique correspondant.

8. Procédé selon l'une des revendications précédentes, **caractérisé en ce que** l'on purifie un acide carboxylique de formule R¹C(O)OH ou que l'on utilise un chlorure d'acide carboxylique de formule R¹C(O)Cl (I) ou R¹C(O)CH₂C(O)Cl (II), où R¹ est un alkyle substitué par au moins un atome d'halogène qui comporte 1 à 6 atomes de carbone ; et représente en particulier un polyfluoroalkyle, un perfluoroalkyle ou un polyfluorochloroalkyle comportant 1 à 6 atomes de carbone.

9. Procédé selon la revendication 7, **caractérisé en ce que** l'on utilise un alcool de formule R²OH (II), où R² est un alkyle ou un alcényle comportant 1 à 8 atomes de carbone ; un alkyle ou un alcényle, substitué par au moins un atome d'halogène, et comportant 1 à 8 atomes de carbone ; un phényle, un tolyle ; un benzyle ; un phényle, tolyle ou benzyle substitué par au moins un atome d'halogène et/ou au moins un groupe nitro.

10. Procédé selon la revendication 5, **caractérisé en ce que** l'on purifie ou fabrique un ester de formule (III) R¹C(O)OR² ou (IV) R¹C(O)CH₂C(O)OR², où R¹ et R² ont la signification donnée précédemment.

11. Procédé selon l'une des revendications 8 à 10, **caractérisé en ce que** R¹ est un polyfluoroalkyle, un perfluoroalkyle ou un polyfluorochloroalkyle comportant 1 à 4 atomes de carbone et R² est un alkyle ou un alcényle comportant 1 à 4 atomes de carbone ; un alkyle ou un alcényle substitué par au moins un atome d'halogène et comportant 1 à 4 atomes de carbone ; un phényle ; un phényle substitué par au moins un atome d'halogène et/ou par au moins un groupe nitro.

12. Procédé selon la revendication 11, **caractérisé en ce que** R¹ est un perfluorométhyle, un perfluoroéthyle, un perfluoropropyle ou un chlorodifluorométhyle.

13. Procédé selon la revendication 12, **caractérisé en ce que** R² est un alkyle ou un alcényle comportant 1 à 3 atomes de carbone ; un alkyle ou un alcényle substitué par au moins un atome de fluor et comportant 1 à 3 atomes de carbone ; un phényle ; un phényle substitué par au moins un atome de fluor et/ou au moins un groupe nitro.

14. Procédé selon la revendication 7, **caractérisé en ce que** le rapport molaire entre chlorure d'acide et sel "d'onium" est compris dans la gamme de 1:1 à 20 000:1.

15. Procédé selon la revendication 14, **caractérisé en ce que** l'on utilise un sel de pyridinium ou de pipéridinium.

16. Procédé selon la revendication 7, dans lequel le sel "d'onium" est formé in situ à partir de l'acide carboxylique correspondant et de la base correspondant au cation d'onium.

17. Procédé selon la revendication 16, dans lequel on part, avec des acides carboxyliques tendant à la décarboxylation, d'un acide carboxylique stable vis-à-vis d'une décarboxylation.

18. Procédé selon la revendication 12, **caractérisé en ce que** l'on utilise R¹C(O)CH₂C(O)Cl qui a été obtenu par réaction de R¹C(O)Cl et de cétène.

19. Procédé selon la revendication 1, **caractérisé en ce que** l'on purifie ou prépare CF₃C(O)Cl ou CF₃C(O)CH₂C(O)Cl, ou qu'on l'utilise lors de la dérivation, en particulier de la fabrication d'un ester.

20. Procédé selon la revendication 7 pour fabriquer des esters méthyliques ou éthyliques de l'acide trifluoroacétique et de l'acide chlorodifluoroacétique, dans lequel on fait réagir le chlorure d'acide avec un excès stoechiométrique de l'alcool en présence du sel "d'onium" et de SiO₂ amorphe, et dans lequel le rapport molaire entre alcool et chlorure d'acide est choisi de façon à ce que deux phases se forment, une phase contenant l'ester selon une pureté pouvant être atteinte sans étape de distillation d'au moins 95 % en poids, puis on isole l'ester par séparation de la phase ester de l'autre phase.

21. Procédé selon la revendication 20, **caractérisé en ce que** l'on prépare l'ester méthylique de l'acide trifluoroacétique et que le rapport molaire entre méthanol et chlorure de trifluoroacétyle est compris dans la gamme de 1,03:1 à 4:1.

22. Procédé selon la revendication 20, **caractérisé en ce que** l'on prépare l'ester éthylique de l'acide trifluoroacétique et que le rapport molaire entre éthanol et chlorure de trifluoroacétyle est compris dans la gamme de 1,01:1 à 5:1.

23. Procédé selon la revendication 20, **caractérisé en ce que** l'on prépare l'ester méthylique de l'acide chlorodifluoroacétique et que le rapport molaire entre méthanol et chlorure de chlorodifluoroacétyle est compris dans la gamme de 1,06:1 à 2,5:1.

24. Procédé selon la revendication 20, **caractérisé en ce que** l'on prépare l'ester éthylique de l'acide chlorodifluoroacétique et que le rapport molaire entre éthanol et chlorure de chlorodifluoroacétyle est compris dans la gamme de 1,02:1 à 2,5:1.

25. Procédé selon la revendication 20, **caractérisé en ce que** l'on introduit le mélange réactionnel dans le cycle et qu'on le met en contact avec un adsorbant.

26. Procédé selon la revendication 6, **caractérisé en ce que** l'on utilise des perles de gel de silice dans des systèmes aqueux et que l'on purifie dans le courant de circulation.

27. Procédé selon la revendication 1, **caractérisé en ce que** le rapport molaire entre sel "d'onium" et HF est supérieur ou égal à 1:2.
